(19) Europäisches Patentamt
European Patent Office
Office européen des brevets

(11) Publication number : **0 587 424 A1**

(12) # EUROPEAN PATENT APPLICATION

(21) Application number : **93307091.4**

(22) Date of filing : **08.09.93**

(51) Int. Cl.$^5$ : **C07C 209/60**

(30) Priority : **08.09.92 US 941487**

(43) Date of publication of application : **16.03.94 Bulletin 94/11**

(84) Designated Contracting States : **BE DE FR GB LU NL**

(71) Applicant : **TEXACO CHEMICAL COMPANY
3040 Post Oak Boulevard
Houston, Texas 77056 (US)**

(72) Inventor : **Benac, Brian Lawrence
104 Nichols
San Marcos, Texas 78666 (US)**
Inventor : **Knifton, John Frederick
10900 Catskill Trail
Austin, Texas 78750 (US)**
Inventor : **Dai, Eugene, Pei Sheng Eugene
3437 Brittany
Pt. Arthur, Texas 77642 (US)**

(74) Representative : **Green, Mark Charles et al
Urquhart-Dykes & Lord, 91 Wimpole Street
London W1M 8AH (GB)**

(54) **Alkylamine synthesis over dealuminated y-type zeolites.**

(57) A method for preparation of alkylamines, particularly t-butylamine, which comprises reacting ammonia and a monounsaturated or polyunsaturated olefin having 2 to 10 carbon atoms in the presence of a dealuminate Y-zeolite.

EP 0 587 424 A1

This invention relates to the synthesis of alkylamines. More particularly this invention relates to a method for the synthesis of alkylamines which comprises reacting ammonia with a monounsaturated or polyunsaturated olefin of 2 to 10 carbon atoms, or mixtures thereof, over a catalyst comprising a dealuminated Y-type zeolite at a specified liquid hourly space velocity and temperature and pressure range. For instance, ammonia can be reacted with isobutylene by the method of this invention to produce t-butylamine with conversions optimized from 12 to 17 (molar) percent.

Surprisingly the olefin conversion level with dealuminated Y-type zeolite catalysts and the related catalysts reported herein, under the specified conditions, approximates the thermodynamically calculated limit. In addition, the catalyst exhibits long life, requires no regeneration and exhibits little coke formation.

Lower aliphatic amines find applications as organic intermediates for the synthesis of drugs, and also e.g. as bactericides, herbicides, rubber accelerators, corrosion inhibitors, extraction agents in the production of penicillin and surface active agents.

Previously morpholine has been used as a rubber accelerator in various processes. Recently in the art there has been a trend to use primary amines as alternatives for morpholine. It has been demonstrated that highly hindered primary amines such as t-butylamine are especially useful in this regard.

Methods of preparation of primary amines are known in the art. "Functionalisation of Alkenes: Catalytic Amination of Monolefins", by J.J. Brunet et al., J. Mol. Catal., 49 (1989) 235-259 presents a review of amination of monoolefins.

The most widespread method for producing lower aliphatic amines is a reaction first achieved by Sabatier, involving the reaction of ammonia and an alcohol. Catalysts which have been used for the Sabatier reaction include silica-alumina, binary transition metal oxides, various metal phosphates and, recently, zeolites. Processes involving the reaction of ammonia and alcohols produce water which has to be separated from the product amine. There has long been a desire in the art to manufacture amines directly from alkenes, since it would save a step in commercial operations.

The direct addition of ammonia to alkenes is thermodynamically feasible, at least for ethylene and propylene, and can be accomplished in two ways, either by activation of the olefinic bond by coordination on a metal complex, thus forming a new species which can undergo nucleophilic attack by activation of the amine, or by activation of the amine by making it either more electrophilic(via amino radicals) or more nucleophilic(via metal amides).

Activation of an olefin can be promoted by many transition metals. However, none of the products are generally the result of a simple monoaddition of the nucleophile on the olefin.

Markovnikov-addition-type monoamines have been formed by vapor phase amination of monoolefins in the presence of aluminosilicates, see US-A-4,307,250 (1981) and US-A-4,375,002 (1983) to J.O.H. Peterson et al. Highest activities were obtained with small to medium pore acidic zeolites such as H-eronite and H-clinoptilotite, which were more effective than standard Linde SK-500 zeolite, a rare earth-exchanged Y-zeolite. These catalysts exhibit higher selectivity at high temperature, thus decreasing the chance of ethylene polymerization and other side reactions. In this work, stoichiometric excess of ammonia appeared to contribute to high selectivities to monoethylamine.

Similar zeolite catalysts have been used by others to effect ethylene amination to ethylamine. An article titled "Direct Amination of Ethylene by Zeolite Catalysis" by M. Deeba, M.E. Ford and T.A. Johnson, J. Chem. Soc. Commun., 1987, 562, describes the formation of ethylamine by addition of ammonia to ethylene catalyzed by acidic zeolites such as H-Y, H-mordenite and H-erionite.

The characteristic which allows these zeolites to act as catalysts for ethylene amination again is thought to result from the highly acidic nature of the proton-exchanged zeolites. The necessity of strongly acidic sites and, thus, of a protonated ethylene intermediate for catalytic amination is demonstrated by the negligible activity of the non-acidic, sodium ion-exchanged offretite and Y-zeolites and the weakly acidic amorphous silica alumina. In addition Johnson reported a high selectivity that was believed to be the result, of a stoichiometric excess of ammonia, Ibid.

The same researchers reported further work in "Amination of Olefins by Zeolites" by Deeba et al., Catalysis Today, 1987, 221. H-offretite, H-erionite, H-clinoptilolite, H-Y and rare earth-exchanged Y-zeolites were employed to obtain selectivities to the corresponding amines as high as 97%. This work included the conversions of ethylene, propylene and isobutylene to the corresponding amines. Isobutylene was selectively converted to t-butylamine with greater than 98% yield between 220°C and 300°C. It was noted that there was no detectable conversion of isobutylene with small pore zeolites such as H-erionite or H-clinoptilolite.

The activity of the zeolites investigated was correlated to the number of strong acid sites as determined by ammonia TPD.

It is also indicated that the mechanism of olefin amination results from the highly acidic nature of proton-exchanged zeolites, wherein ethylene and propylene are reversibly adsorbed by zeolites, probably by means

of the formation of a pi complex between the surface hydroxy group and the olefin.

It was also concluded that the conversion of ethylene required the highest temperatures and the strongest acid sites, while propylene formed a more stable cationic intermediate activated by somewhat weaker acid sites.

In the case of isobutylene amination by ammonia, calculated product distributions indicate amination is favored by low temperature, high pressure and high ammonia/isobutylene ratios.

In J.Org. Chem., 53, 4594 (1988), M. Deeba and M.E. Ford report their first attempt to extend information regarding the acid-catalyzed amination of ethylene to ethylamine via zeolite catalysis to the preparation of t-butylamine from isobutylene which was believed to be limited by thermodynamic equilibrium. They suggest high ammonia-to-isobutene ratios favor the reaction. Conversion to t-butylamine with zeolite catalysts passes through a maximum at 260°-280°C. These authors found that H-mordenite displayed lower activity than either H-Y or rare earth exchanged Y-zeolites.

Modified zeolites have also been used for producing amines. In U.S. Patent 4,929,759, Taglieber et al. disclose a process for the preparation of an amine from an olefin and ammonia in the presence of a borosilicate or borogermanate zeolite of the pentasil type at a temperature of 80°C to 400°C and a pressure of between about 3.5 and about 76 MPa (about 500 and about 11,000 psig). In US-A-4,929,758, Taglieber et al. disclose the preparation of tert-butylamine from isobutylene and ammonia in the presence of an iron silicate catalyst of the pentasil type. (See also German Patents 3327-000A and 3634-247C to BASF.)

Zeolite catalysts were also used for preparation of amines by V. Taglieber et al., CA-A-1,218,677 (1987) to BASF. In this process, ammonia and amines are mixed with the olefin and reacted from about 2 x $10^7$ to 3 x $10^7$ Pa and about 250° to 350°C. The catalysts were zeolites of the pentasil type. Pentasils are generally medium-pore zeolites having a predominance of silicon.

US-A-4,302,603 (1981), to G. Pez, disclosed that a substantial improvement is obtained when the direct reaction of a monoolefin with ammonia is homogeneously catalyzed in liquid ammonia by $CsNH_2$, $RbNH_2$, or mixtures which may also include $KNH_2$ or $NaNH_2$ at a temperature below the critical temperature of ammonia.

In US-A-937,383 to Brigandat et al. (1990), there is disclosed a process for the manufacture of amines by reacting an olefin and ammonia in the presence of a catalyst comprising ammonium sulfate or a double sulfate of ammonium and a transition metal.

Most recently, in an article in Chem. Lett., 1027 (1991), M. Tabata , N. Mizuno and M. Iwamoto report that, although direct amination of ethene to ethylamine has been achieved using alkali metal catalysts in a homogeneous system, these systems seem to actually provide low yields of higher alkylamines. These authors demonstrate the formation of t-butylamine directly from 2-methylpropene catalyzed by a proton-exchanged ZSM-5 zeolite. The authors noted a variation in activity among the zeolite catalysts used and they concluded the controlling factor was the Bronsted acidity of the catalyst.

It is apparent from the art that attempts to produce t-butylamine from isobutylene by reacting alkylenes with ammonia have not been completely successful, despite the fact that good yields of ethylamine have generally been demonstrated. In addition, the art would indicate that in any attempt to produce t-butylamine high stoichiometric ratios of ammonia to olefins are required.

In the few attempts reported in the art where the goal of researchers was to aminate isobutylene using a zeolite catalyst, zeolites were employed which were high in alumina and which typically had silica-to-alumina molar ratios close to unity.

In instances where there appeared to be some degree of success it seemed to be attributed to the acidity of the zeolite.

However, where t-butylamine has been produced, the selectivities did not approach quantitative and formation of olefin oligomers was often a problem. Furthermore, the useful life of such an amination catalyst does not appear to have been addressed in any reported work.

It would be a distinct advance in the art if a catalyst were available for converting isobutylene to t-butylamine which exhibited a long useful life, gave conversions approaching the thermodynamically calculated limit, allowed for selectivities close to quantitative, suppressed formation of olefin oligomers, and permitted rapid amination with stoichiometric quantities of ammonia.

According to the present invention, there is provided a process for the production of primary alkylamines which comprises reacting ammonia with a monounsaturated or polyunsaturated olefin having 2 to 10 carbon atoms, or mixtures thereof, in the presence of a catalyst consisting essentially of a dealuminated Y-zeolite. The dealumination may be effected by ammonium exchange, followed by calcination, chelation of the alumina by ethylenediaminetetraacetic acid or treatment with fluorine compounds or hydrothermal and/or acid treatment.

In a preferred method according to the present invention, ammonia is reacted with a monounsaturated olefin of 2 to 10 carbon atoms, or mixtures thereof, in the presence of a catalyst comprising a dealuminated Y-zeolite at a temperature in the range from 100°C to 350°C, a pressure of from 7 to 35 MPa (from 1000 to

5000 psig), a space velocity of 0.1 to 10 and a molar ratio of alkylene to ammonia of 1:1 to 1:10.

The invention is particularly effective in the reaction of ammonia with isobutylene to synthesize tert-butylamine. This particular reaction can be represented by:

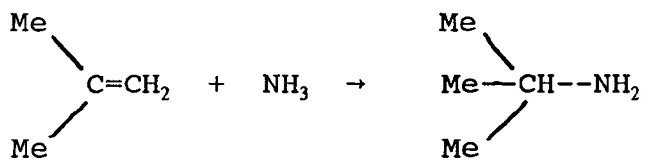 (Eq. 1)

Starting materials employed in the process are olefins containing 2 to 10 carbon atoms and ammonia. The olefin is preferably an aliphatic linear, or branched-chain, monounsaturated olefin having from 2 to 6 carbon atoms per molecule, such as ethylene, propylene, isobutylene and isoamylene. The most preferred olefin is isobutylene.

In the amination of the olefins, ammonia or an ammonia type compound, i.e., a primary or secondary amine can be used as the reactant. Suitable primary and secondary amines can be represented by the formula $RNH_2$ and $(R)_2NH$ where each R is a hydrocarbon group having from 1-6 carbon atoms in the structure. Examples of amines include methylamine, dimethylamine, mono and di-n-propylamine, cyclohexylamine and the like. Lower alkyl amines are preferred. In the instant invention the preferred reactant was ammonia.

Ammonia, along with the olefin, is fed into a pressure reactor fitted with means of mixing in streams adjusted to maintain a mole ratio of ammonia to olefin of between 1:1 and 10:1. The reaction is preferably maintained at a temperature of from 100° to 350°C and a pressure of 7 to 24 MPa (1000-3500 psig) and the ammonia and olefin are passed over the catalyst producing as effluents alkylamines and unconverted ammonia and olefin. Typical samples of product effluent were collected in on-line bombs for one hour and analyzed by gas chromatography.

The catalysts suitable for the practice of this invention generally comprise dealuminated Y-zeolite catalysts.

There are a number of methods known in the art for dealuminating zeolites. A reference which provides an informative overview of the various processes is "Catalytic Materials:Relationship between Structure and Reactivity", Ed. White, T. E. et al., Ch. 10, American Chemical Society, Washington, D.C., 1984. (Based on the 1983 State-of-the-Art Symposium sponsored by the Division of Industrial, and Engineering Chemistry, San Francisco, California, June 13-16, 1983.)

Each method of dealumination results in a framework modified to a different extent. The resulting zeolite can be not only dealuminized, but in some cases structurally rearranged.

Only zeolites dealuminized by particular methods would produce a zeolite catalyst which provides the desired results in tert-butylamine synthesis.

The methods of dealumination which provide the preferred structure in the resulting framework are produced by:

a) Ammonium exchange, followed by calcination;

b) Chelation of alumina by treatment with EDTA;

c) Treatment of the zeolite with fluorine or a fluorine-containing reactant; and

d) Hydrothermal and/or acid treatment.

The preferred catalysts for use in the dealuminated form for the reaction of Eq. 1 are certain crystalline aluminosilicate zeolites, particularly the isostructural group of faujasite zeolites that include the synthetic X- and Y-zeolites. The preferred zeolites for dealumination are the Y-zeolites.

The unit cells of faujasite zeolites are cubic, $a_o \approx 2.5$ nm, and each contains 192 silicon- or aluminum-centered oxygen tetrahedra which are linked through shared oxygen atoms. Because of the net negative charge on each of the aluminum-centered tetrahedra, each unit cell contains an equivalent number of charge-balancing cations. These are exclusively sodium ions in zeolites in their synthesized form. Typical cell contents for the Y-zeolites in the hydrated form are:

$$Na_{56}[(AlO_2)_{56}(SiO_2)_{136}]_x 250\ H_20$$

Y-zeolites are distinguished on the basis of the relative concentration of silicon and aluminum atoms and the consequent effects on detailed structure and related chemical and physical properties. The aluminum atoms in the unit cell of Y-zeolite vary from 76 to 48, resulting in a Si:Al ratio between 1.5 and 3.0. Both the cation concentration and charge density on the aluminosilicate structure are lower for Y-zeolites than for X-zeolites, where the aluminum atoms in the unit cell vary from 96 to 77.

The feature which determines the difference between faujasites and other zeolites built up from sodalite units is the double 6-membered ring or hexagonal prism, by which the units are linked. The sodalite unit, or β-cage, can be represented by a truncated octahedron, with the 24 silicon or aluminum atoms(designated T atoms) taking positions at the vertices. The 36 oxygen atoms are displaced from the midpoints of the edges joining the vertices in order to attain tetrahedral configuration around the T atoms. The free diameter of the void within the β-cage is 0.66 nm, but only the smallest molecules can enter through the 0.22 nm diameter opening in the distorted ring of six oxygen atoms associated with each hexagonal face. Each sodalite unit is linked tetrahedrally across hexagonal faces by six bridging oxygens to four other sodalite units. The larger void spaces enclosed by sodalite units and hexagonal prisms are termed α-cages, or supercages. The α-cage is a 26-hedron with a free diameter of ≈ 1.3 nm, and it can be entered through four distorted 12-member rings of diameter 0.80-0.90 nm. In this way each a-cage is tetrahedrally joined to four others giving a complex system of void space extending throughout the zeolite structure. The α- and β-cages together give Y-zeolites, along with X-zeolites, the largest void volume of any known zeolites, which is ca. 50 vol% of the dehydrated crystal. From the catalytic viewpoint, the α-cages are by far the most important, since, unlike the β-cages, they permit entry of numerous aliphatic and aromatic compounds.

It has been demonstrated in the instant invention these Y-zeolites are particularly effective in the dealuminated form. The Y-zeolites are dealuminated by ammonium exchange followed by calcination, or by treatment with ethylenediaminetetraacetic acid (EDTA) or other chelating agents or by treatment with fluorine or hydrothermal treatment and/or acid treatment. Said dealuminated Y-zeolites should have a silica-to-alumina molar ratio of greater than three, preferably a ratio of 5 or greater and most preferably a silica-to-alumina ratio of 5 to 100. The examples demonstrate the usefulness of catalysts having a silica-to-alumina ratio of 5 to 50 with the majority in the range of 5 to 25 and particularly 5 to 10.

A variety of methods of dealumination are known in the art and are discussed in "Catalytic Materials", Ch. 10, ibid., p. 158.

For example, dealumination can be accomplished by thermal treatment of an ammonium zeolite under steam. In the resulting zeolite defective sites left by alumination are filled to a large extent by silica, which leads to a very stable zeolite.

Using a chelating agent such as EDTA, up to about 50% of aluminum atoms can be removed from the zeolite in the form of a water soluble chelate, without any appreciable loss in zeolite crystallinity and about 80% of aluminum atoms can be removed while the zeolite maintains 60% to 70% of its crystallinity.

The fluorine gas treatment can be carried out using a dilute $F_2$ stream (<20%) at temperatures from ambient to about 100°C and brief contact times of preferably less than 10 minutes. Another approach involves solution phase treatment with silicon fluoride salts such as $(NH_4)_2SiF_6$ or $Li_2SiF_6$. Using silicon fluoride salts the framework vacancies can be refilled with Si. Ibid, p. 162. US-A-4,503,023 teaches the procedure for treating zeolites with $(NH_4)_2SiF_6$.

Finally, a combination of hydrothermal and/or acid treatment can be used to treat zeolites, ibid, p. 162.

Two other reports, the circulation of which is restricted are:

"Advances in Catalytic Process Technologies: Zeolite and Molecular Sieves," Study No. 4186CAI, 1987; and "New Catalytic Materials: Volume VI Advances in Zeolite Technology," Study No. 41832, May 1984, prepared by :

Catalytica®
430 Ferguson Drive, Building 3
Mountain View, California 94043

They might be procured by written request.

Examples of suitable commercially available dealuminized Y-zeolites include UOP's LZY-82 and LZY-72, PQ Corporation's CP-304-37 and CP-316-26, UOP's Y-85, Y-84, LZ-10 and LZ-210.

The unit cell size and $SiO_2/Al_2O_3$ molar ratio for dealuminated Y-zeolites is noted in the following table:

| ZEOLITE TYPE | UNIT CELL SIZE, A | $SiO_2/Al_2O_3$MOLAR |
|---|---|---|
| LZY-82 | 24.53 | 7.8 |
| LZY-85 | 24.49 | 9.1 |
| LZY-10 | 24.32 | 23.7 |
| LZY-20 | 24.35 | 18.9 |
| LZY-84 | 24.51 | 8.4 |
| LZ-210 | 24.47 | 9.9 |
| LZY-72 | 24.52 | 8.1 |
| CP316-26 | 24.26 | 45.7 |

The examples demonstrate the particular effectiveness of LZY-82, having a silica-to-alumina molar ratio of 7.8 which has been dealuminated by ammonium exchange followed by calcination.

The related art would indicate that in the case of conversion of isobutylene, high stoichiometric amounts of ammonia are required. Where the ammonia/isobutylene feed ratio drops below 1/1 mole ratio, the acid-catalyzed polymerization of isobutylene intervenes and selectivity to tert-butylamine decreases rapidly. In the instant invention the molar ratio of ammonia or amine to olefin is optionally 2:1 but can range from 1:1 to 10:1.

The reaction may be carried out in either a stirred slurry reactor or a fixed bed continuous flow reactor. The catalyst concentration should be sufficient to provide the desired catalytic effect.

Amination can generally be conducted at temperatures from 100°C to 350°C; the preferred range is 200° to 320°C and the most preferred range is 260° to 300°C. The total operating pressure range is 7 MPa to 34.6 MPa (1000 psig to 5000 psig), the preferred range is 10.4 MPa to 24.2 MPa (1500 to 3500 psig).

The reaction can take place in an upflow or downflow reactor at liquid hourly space velocities ranging from 0.1 to 10.0. Typical primary alkylamines such as t-butylamine are generated continuously in up to 25 wt% concentration in the crude liquid product at a total liquid hourly space velocity (LHSV) of up to 5 or higher. LHSVs of 0.2 to 2.0 have been demonstrated as particularly useful in achieving the desired alkylene conversion.

$$LHSV = \frac{\text{Volume of Total Liquid Feed Run Through the Reactor Per Hour}}{\text{Volume of Catalyst in Reactor}}$$

The Examples which follow illustrate the one-step synthesis of alkylamines, such as t-butylamine, from olefins containing 2-10 carbon atoms and ammonia, using the dealuminated Y-type zeolites, particularly those dealuminized Y-zeolites having a silica-to-alumina molar ratio in the range 5 to 100. The conversion of isobutylene is as high as 17% and the selectivity for tert-butylamine reaches 99% or greater.

Conversion of olefins to t-butanol (TBA, wt%) are estimated in the following examples using the formula:

$$\frac{\text{(Wt\% Conc. of Alkylamine in Product} - \text{Wt\% Conc. TBA in Feed)}}{\text{Wt\% Conc. of TBA in Feed}} \times 100$$

Selectivities of alkylamine are estimated from:

$$\frac{\text{Moles of Alkylamine in Product}}{\text{Moles of Isobutylene Converted}} \times 100$$

The following examples are intended only as a means of illustration and are not intended to limit the invention in any way.

| Ex. No. | Catalyst | LHSV | Pressure* (psig) | Direction of Flow |
|---|---|---|---|---|
| 1† | SK-500 | 1 | 1500 | Up |
| 2 | LZY-72 | 1 | 1500 | Up |
| 3 | LZY-82 | 1 | 1500 | Up |
| 4 | USY | 1 | 1500 | Up |
| 5 | LZY-72 | 1 | 1500 | Up |
| 6 | LZY-84 | 1 | 1500 | Up |
| 7 | LZ-10 | 1 | 1500 | Up |
| 8 | LZ-20 | 1 | 1500 | Up |
| 9 | CP316-26 | 1 | 1500 | Up |
| 10 | LZY-82 | 0.5 | 2500 | Up |
| 11 | LZY-82 | 1.0 | 2500 | Up |
| 12 | LZY-82 | 2.0 | 2500 | Up |
| 13 | LZY-82 | 1.0 | 2500 | Down |
| 14 | LZY-82 | 0.5 | 1500 | Up |
| 15 | LZY-82 | 0.5 | 3500 | Up |
| 16 | LZY-82 | 1 | 1500 | Up |
| 17 | LZY-82 | 1 | 1500 | Up |

* 1500 psig = 10.4 MPa
2500 psig = 17.3 MPa
3500 psig = 24.2 MPa

† Comparative Experiment

## COMPARATIVE EXAMPLE 1

This Comparative Example illustrates the performances of a rare-earth exchanged Y-zeolite in the synthesis of t-butylamine from isobutylene plus ammonia.

A temperature scan experiment was performed using UOP SK-500, a lanthanide exchanged Y-type zeolite. A 100cc, upflow, electrically heated reactor was filled with the catalyst. A feed composed of a 2:1 molar ratio of ammonia to isobutylene was used at a total liquid hourly space velocity of 1 at 10.4 MPa (1500 psig). The temperature scan was performed from 200 to 320°C in 20°C increments. Typically flow of the ammonia/isobutylene feed was initiated and the reactor was heated to the required temperature, controlling on the central thermocouple which was measuring the skin temperature of the reactor. Once the correct temperature had been reached the system was allowed to come to equilibrium for 4 hours and then a sample of the reactor effluent was collected for 1 hour. The sample was then analyzed by gas chromatography using a method that gave wt% ammonia, isobutylene, t-butylamine and any diisobutylene. From these results isobutylene to t-butylamine conversion levels were calculated. Typical results were 8.3% isobutylene conversion at 260°C, 11.1% at 280°C and 10.8% at 300°C with better than 99% selectivity to t-butylamine at all three temperatures.

## EXAMPLE 2

This Example illustrates the use of an ammonium-exchanged, dealuminated Y-zeolite having a silica-to-alumina ratio of 8.1, in the synthesis of t-butylamine.

Following the procedures of Example 1, t-butylamine synthesis was demonstrated using UOP's LZY-72. Typical results were 12.1% isobutylene conversion at 260°C, 7.7% at 280°C and 7.7% at 300°C. Better than 99% selectivity to t-butylamine was achieved at 260° and 280°C while there was 93.2% selectivity at 300°C.

## EXAMPLE 3

Similar to Example 1 except that UOP LZY-82 having a silica/alumina ratio of 7.8 was used as the catalyst. Typical results were 13.4% isobutylene conversion at 260°C, 15.7% at 280°C and 8.9% at 300°C. Better than 99% selectivity to t-butylamine was achieved at 260° and 280°C while there was 93.8% selectivity at 300°C.

**EXAMPLE 4**

Similar to Example 1 except that UOP Ultra-Stable Y having a silica/alumina ratio of 9.9 was used as catalyst. Typical results were 3.7% isobutylene. conversion at 260°C, 8.4% at 280°C and 6.3% at 300°C. Better than 99% selectivity to t-butylamine was achieved at 260° and 280°C while there was 86.8% selectivity at 300°C.

**EXAMPLE 5**

This Example also used UOP LZY-72 as the catalyst. Typical results were 4.6% isobutylene conversion at 260°C, 8.2% at 280°C and 7.1% at 300°C. Better than 99% selectivity to t-butylamine was achieved at 260° and 280°C while there was 92.3% selectivity at 300°C.

**EXAMPLE 6**

Similar to Example 1 except that UOP LZY-84 having a silica-to-alumina ratio of 8.4 was used as the catalyst. Typical results were 5.3% isobutylene conversion at 260°C, 10.5% at 280°C and 8.1% at 300°C. Better than 99% selectivity to t-butylamine was achieved at 260° and 280°C while there was 90.9% selectivity at 300°C.

**EXAMPLE 7**

Similar to Example 1 except that UOP LZ-10 having a silica-to-alumina ratio of 23.7 was used as the catalyst. Typical results were 4.9% isobutylene conversion at 260°C, 6.7 at 280°C and 8.5% at 300°C. Better than 99% selectivity to t-butylamine was achieved at 260° and 280°C while there was 88.1% selectivity at 300°C.

**EXAMPLE 8**

Similar to Example 1 except that UOP LZ-20 having a silica-to-alumina ratio of 18.9 was used as the catalyst. Typical results were 5.8% isobutylene conversion at 260°C, 4.1% at 280°C and 6.3% at 300°C. Better than 99% selectivity to t-butylamine was achieved at 260° to 280°C while there was 90.0% selectivity at 300°C.

**EXAMPLE 9**

Similar to Example 1 except that PQ Corp. CP 316-26 having a silica/alumina ratio of 45.7 was used as the catalyst. Typical results were 3.4% isobutylene conversion at 260°C, 6.6% at 280°C and 4.1% at 300°C. Better than 99% selectivity to t-butylamine was achieved at all three temperatures.

**TABLE 1**

Isobutylene Conversion (%) versus Temperature for Y-Type Zeolites at 10.4 MPa (1500 psig) and LHSV of 1

| Catalyst | 260 | Temperature (°C) 280 | 300 |
|---|---|---|---|
| UOP SK-500 | 8.3 | 11.1 | 10.8 |
| UOP LZY-72 | 12.1 | 7.7 | 7.7 (92.3) |
| UOP LZY-82 | 13.4 | 15.7 | 8.9 (93.8) |
| UOP Ultra Stable Y | 3.7 | 8.4 | 6.3 (86.8) |
| UOP LZY-84 | 5.3 | 10.5 | 8.1 (90.9) |
| UOP LZ-10 | 4.9 | 6.7 | 8.5 (88.1) |
| UOP LZ-20 | 5.8 | 4.1 | 6.3 (90.0) |
| PQ Corp. CP 316-26 | 3.4 | 6.6 | 4.1 |

Unless listed in parentheses next to Isobutylene Conversion, all t-butylamine selectivities are greater than 99%.

## EXAMPLE 10

A temperature scan experiment was performed using a 550cc, Dowtherm® heated, upflow reactor filled with 400cc of dried UOP LZY-82 and 75cc of 6mm glass beads at the bottom and top of the reactor. Ammonia and isobutylene were pumped separately and mixed in an approximate 2:1 molar ratio of ammonia to isobutylene composition prior to being admitted to the reactor at a total liquid hourly space velocity of 0.5 and 2500 psig. The temperature scan was performed at 220°C and then from 240° to 320°C in 10°C increments. Typically, flow of the ammonia feed was initiated and the reactor was heated to 100°C before the isobutylene feed was started. The reactor was then heated to the required temperature while controlling on the central thermocouple which was measuring the temperature inside the reactor. Once the correct temperature had been reached the system was allowed to come to equilibrium for 4 hours and then a sample of the reactor effluent was collected for 1.5 hours. The sample was then analyzed by gas chromatography using a method that gave the wt% of ammonia, isobutylene, t-butylamine and any diisobutylene. From these results isobutylene to t-butylamine conversion levels were calculated. Typical results were 7.9% isobutylene conversion at 260°C 12.2% at 280°C and 11.3% at 300°C. Better than 99% selectivity to t-butylamine was achieved at 260° and 280°C while there was 95.8% selectivity at 300°C.

## EXAMPLE 11

Similar to Example 10 except that a total liquid hourly space velocity of 1.0 was used. Typical results were 6.6% isobutylene conversion at 260°C, 14.1% at 280°C and 10.8% at 300°C. Better than 99% selectivity to t-butylamine was achieved at 260° and 280°C while there was 96.3% selectivity at 300°C.

## EXAMPLE 12

Similar to Example 10 except that a total liquid hourly space velocity of 2.0 was used. Typical results were 2.2% isobutylene conversion at 260°C, 10.0% at 280°C and 10.2% at 300°C with better than 99% selectivity to t-butylamine at all three temperatures.

| TABLE 2<br>Effect of Space Velocity and Temperature on Isobutylene Conversion to t-Butylamine Using UOP LZY-82 at 17.3 MPa (2500 psig) | | | |
|---|---|---|---|
| Space Velocity (cc/cc/-cathr) | Temperature (°C)<br>260 | 280 | 300 |
| 0.5 | 7.9 | 12.2 | 11.3 (95.8) |
| 1.0 | 6.6 | 14.1 | 10.8 |
| 2.0 | 2.2 | 10.0 | 10.2 |
| Unless listed in parentheses next to Isobutylene Conversion, all t-butylamine selectivities are greater than 99%. | | | |

## EXAMPLE 13

The experimental setup was similar to Example 10 except that the reactor was engineered to run downflow. The ammonia and isobutylene (2:1 molar ratio) were fed at a total liquid hourly space velocity of 1.0 at 17.3 MPa (2500 psig). The temperature scans went from 240° to 320°C in 10°C increments. Typical results were 5.7% isobutylene conversion at 260°C, 15.1% at 280°C and 9.6% at 300°C. Better than 99% selectivity to t-butylamine was achieved at 260° and 280°C while there was 96.0% selectivity at 300°C.

| TABLE 3<br>Effect of Direction of Feed Flow and Temperature on Isobutylene Conversion to t-Butylamine using UOP LZY-82 at 17.3 MPa (2500 psig) and LHSV = 1.0 | | | |
|---|---|---|---|
| Direction of Flow | Temperature (°C)<br>260 | 280 | 300 |
| Up | 6.6 | 14.1 | 10.8 (96.3) |
| Down | 5.7 | 15.1 | 9.6 (96.0) |
| Unless listed in parentheses next to Isobutylene Conversion, all t-butylamine selectivities are greater than 99%. | | | |

## EXAMPLE 14

Similar to Example 10 except that the temperature scan was from 240° to 300°C in 10°C increments and the pressure of the reactor was 10.4 MPa (1500 psig). Typical results were 2.2% isobutylene conversion at 260°C, 10.0% at 280°C and 10.2% at 300°C. Better than 99% selectivity to t-butylamine was achieved at 260° and 280°C while there was 68.8% selectivity at 300°C.

## EXAMPLE 15

Similar to Example 10 except that the temperature scan was from 240° to 280°C in 10°C increments and the pressure of the reactor was 24.2 MPa (3500 psig). Typical results were 8.3% isobutylene conversion at 260°C and 17.1% conversion at 280°C with better than 99% selectivity to t-butylamine at both those temperatures.

TABLE 4

Effect of Pressure and Temperature on Isobutylene Conversion to t-Butylamine using UOP LZY-82 and LHSV = 0.5

| Pressure (psig) (MPa) | Temperature (°C) 260 | 280 | 300 |
|---|---|---|---|
| 1500 10.4 | 8.4 | 9.7 | 6.0 (68.8) |
| 2500 17.3 | 7.9 | 12.2 | 11.3 (95.8) |
| 3500 24.2 | 8.3 | 17.1 | NA |

Unless listed in parentheses next to Isobutylene Conversion, all t-butylamine selectivities are greater than 99%.

## EXAMPLE 16

This experiment was set up similar to Example 1 except that UOP LZY-82 was the catalyst. The lifetime experiment was run at 275°C exclusively at a LHSV of 1 using an ammonia/isobutylene (2:1 molar ratio) feed. The last sample analyzed had a 9.7% isobutylene conversion with greater than 99% selectivity to t-butylamine. This run lasted 430 hours with the average isobutylene conversion being 10.5% with selectivities always greater than 99%.

## EXAMPLE 17

This experiment was set up similar to Example 1 except that UOP LZY-82 was the catalyst. The lifetime experiment was run at 275°C at a LHSV of 1 using an ammonia/isobutylene (2:1 molar ratio) feed. The last sample analyzed had an isobutylene conversion of 10.5% with greater than 99% selectivity to t-butylamine. This run lasted 2053 hours with the average isobutylene conversion being 9.6% with selectivities always greater than 99%.

## EXAMPLE 18

Using the procedures and equipment of Example 1, the generation of t-butylamine from isobutylene plus ammonia was demonstrated using a sample comprising 60% LZY-84, 20% S-115 silicalite and 20% alumina, in 1.6mm (1/16") diameter extruded form, which had been previously treated with a solution of ammonium fluorosilicate, $(NH_4)SiF_6$, then calcined at ca. 540°C for 4 hours. The Si/Al ratio of the catalyst was determined to be 20.3. Typical amination results were 3.6% isobutylene conversion at 260°C and 5.6% isobutylene conversion at 280°C. t-Butylamine selectivity was >99% in both experiments.

## Claims

1. A method for the preparation of an alkylamine comprising reacting ammonia or an amine and an olefin containing 2 to 10 carbon atoms per molecule, in a molar ratio of from 1:1 to 10:1, characterised by the use of a catalyst comprising a Y-zeolite which has been dealuminated by
   a) ammonium exchanging the Y-zeolite followed by calcinating;
   b) chelating the alumina with ethylenediaminetetraacetic acid;
   c) treating the Y-zeolite with silicon tetrafluoride and/or ammonium fluorosilicate;
   d) treating the Y-zeolite with steam alone;
   e) treating the Y-zeolite with acid alone; or
   f) treating the Y-zeolite with steam followed by acid

   at a temperature of 100°C to 350°C and a pressure of 7 to 35 MPa and a liquid hourly space velocity of 0.1 to 10.0.

2. A method as claimed in Claim 1 wherein the olefin is isobutylene.

3. A method as claimed in Claim 1 or Claim 2 wherein the dealuminized Y-zeolite has a silica-to-alumina

molar ratio of 5 or greater.

4. A method as claimed in any one of Claims 1 to 3 wherein the molar ratio of ammonia or amine to olefin is 2:1.

5. A method as claimed in Claim 1 for preparing t-butylamine, achieving greater than 90% selectivity which comprises:
reacting isobutylene with ammonia in a molar ratio of 1:2 in the presence of a dealuminated Y-zeolite at a temperature of 260°C to 300°C, a pressure of 1500 to 3500 psig and a liquid hourly space velocity of 0.2 to 2.0.

6. A method as claimed in Claim 5 wherein the dealuminated Y-zeolite has a silica-to-alumina molar ratio in the range 5 to 100 and a unit cell size in the range 24.2 to 24.6A.

7. A method as claimed in Claim 5 wherein the dealuminized Y-zeolite has a silica-to-alumina ratio of 7.8 and a unit cell size of 24.53A.

8. A method as claimed in Claim 5 wherein the dealuminated Y-zeolite comprises over 50% of the catalyst composition and the remainder is divided between S-115 silicalite and alumina.

European Patent Office

# EUROPEAN SEARCH REPORT

Application Number
EP 93 30 7091

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 305 564 (AIR PRODUCTS AND CHEMICALS INC.)<br>* page 2, line 35 - line 47; page 3, line 15 - page 4, line 5; example 1, table IV - VIII, page 8, line 47 - line 52, claims *<br>--- | 1-5 | C07C209/60 |
| Y | EP-A-0 469 719 (TEXACO CHEMICAL COMPANY)<br>* page 3, line 44 - page 4, line 17; page 8, line 8 - line 55, table 1; claims 1 - 5 *<br>--- | 1-5 | |
| A | US-A-4 512 961 (J. SCHERZER)<br>* column 1, line 53 - line 60; column 3, line 51 - line 54; table I *<br>--- | 6,7 | |
| A | EP-A-0 180 983 (AIR PRODUCTS AND CHEMICALS INC.)<br>* page 3, line 18 - page 5, line 14; claims *<br>--- | 1-5 | |
| A,D | EP-A-0 039 918 (AIR PRODUCT AND CHEMICALS INC.)<br>* page 6, line 23 - page 7, line 8; claims; tables 3-5 *<br>--- | 1-5 | TECHNICAL FIELDS SEARCHED (Int.Cl.5)<br>C07C |
| A,D | JOURNAL OF ORGANIC CHEMISTRY<br>vol. 53 , 1988 , EASTON US<br>pages 4594 - 4596<br>M. DEEBA, M. E. FORD 'Heterogeneous acid-catalyzed amination of isobutene to tert.-butylamine'<br>---<br>-/-- | 1-5 | |

The present search report has been drawn up for all claims

| Place of search | Date of completion of the search | Examiner |
|---|---|---|
| THE HAGUE | 24 November 1993 | Seufert, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 93 30 7091

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | CHEMICAL ABSTRACTS, vol. 100, no. 14, 2 April 1984, Columbus, Ohio, US; abstract no. 105486z, TOLKACHEVA L N ET AL 'Hydration of ethylene and isobutylene on high-silica zeolites' page 113 ; * abstract * & NEFTEKHIMIYA vol. 23, no. 6 , 1983 pages 803 - 7 | 1 | |
| E | WO-A-93 17995 (AKZO) * page 11, catalyst A; table 1, ex 1 -- 4; table 2; claims 1, 10 - 13, 18, 19, 22, 24, 27 - 33 * | 1-5 | |

TECHNICAL FIELDS SEARCHED (Int.Cl.5)

The present search report has been drawn up for all claims

| Place of search | Date of completion of the search | Examiner |
|---|---|---|
| THE HAGUE | 24 November 1993 | Seufert, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)